# EUROPEAN PATENT APPLICATION

(11) **EP 0 780 089 A1**
(43) Date of publication of application: **25.06.1997**
(21) Application number: 96120189.4
(22) Date of filing: 16.12.1996
(51) Int. Cl.: A61B 10/00

(54) **Device for taking biobsy samples, particularly of soft tissues**

(30) Priority: 18.12.1995 IT MI952663
(71) Applicant: Luppi, Libero, 41037 Mirandola (Modena) (IT)
(72) Inventor: Luppi, Libero, 41037 Mirandola (Modena) (IT)
(74) Representative: Modiano, Guido, Dr.-Ing.

(57) **Abstract**

A device for taking biopsy samples, particularly of soft tissues, which comprises a body (2) which supports a trocar cannula (4) inside which a fixed stylet (5) is provided, the device further comprising an automatic element (10) for the advancement of the trocar cannula (4), which is associated with a syringe-like element to produce suction inside the cannula (4) during the forward translatory motion with respect to the fixed stylet (5).

## Description

The present invention relates to a device for taking biopsy samples, particularly of soft tissues.

It is known that the so-called Menghini technique is currently used to obtain biopsy samples of soft tissues; according to this technique, a trocar cannula, combined with a syringe, produces suction at the tissue to be removed, which is thus inserted in the trocar cannula.

This method permits to obtain tissue samples that have a cylindrical cross-section, obtaining the same amount of tissue that can be obtained by means of guillotine removal devices, but with the possibility of using smaller needles, with consequent considerable advantages from the traumatic point of view.

Conventional devices substantially use a syringe-like element having a trocar cannula inside which there is provided a stylet or mandrin which in practice closes the end of the trocar cannula during the initial penetration step.

Once the exact removal region has been reached, it is necessary to actuate the syringe so as to cause the translatory motion of the mandrin or stylet with respect to the trocar cannula and generate the suction which breaks off the tissue.

These operations are performed manually while the needles are inserted in the patient's body, and therefore said operations are relatively difficult; moreover, traumas can often be produced because of the unwanted movements of the cannula which are performed to move the syringe.

Consequently, there is an extreme inaccuracy in reaching very small masses to be biopsied.

A principal aim of the invention is to eliminate the above drawbacks by providing a device for taking biopsy samples, particularly of soft tissues, which allows to perform actuation without having to manually produce, with a syringe, the suction in the trocar cannula, also simplifying the operations to be performed.

Within the scope of this aim, a particular object of the invention is to provide a device for taking biopsy samples which has a substantially automatic actuation and accordingly does not require particular skill for its use.

Another object of the present invention is to provide a device for taking biopsy samples, particularly of soft tissues, which by virtue of its particular constructive characteristics is capable of giving the greatest assurances of reliability and safety in use.

Another object of the present invention is to provide a device which can be easily obtained starting from commonly commercially available elements and materials and is also competitive from a merely economical point of view.

This aim, these objects, and others which will become apparent hereinafter are achieved by a device for taking biopsy samples, particularly of soft tissues, according to the invention, which comprises a body that supports a trocar cannula inside which a fixed stylet is provided, characterized in that it comprises automatic means for the advancement of said trocar cannula, which is associated with a syringe-like element to produce suction inside said trocar cannula during the forward translatory motion with respect to said fixed stylet.

Further characteristics and advantages of the invention will become apparent from the following detailed description of a preferred but not exclusive embodiment of a device for taking biopsy samples, particularly of soft tissues, illustrated only by way of non-limitative example in the accompanying drawings, wherein:
figure 1 is a schematic sectional view of the device according to the invention, preset for its initial step, namely insertion in the tissue;
figure 2 is a view of the step for the release of the automatic advancement means;
figure 3 is a view of the device after removal has been performed;
figure 4 is a schematic sectional view of the device with a shorter removal stroke than the device shown in the preceding figures;
figure 5 is a schematic perspective view, taken from below, of the supporting slide of the trocar cannula;
figure 6 is a perspective view of the elastic ratchet means which act on the slide;
figure 7 is a sectional view, taken along the plane VII-VII of figure 3;
figure 8 is a sectional view, taken along the plane VIII-VIII of figure 3.

With reference to the above figures, the device for taking biopsy samples, particularly of soft tissues, according to the invention, which is generally designated by the reference numeral 1, comprises a body 2 inside which a slide 3 is slidingly associated.

The slide 3 supports a trocar cannula 4, inside which there is provided a stylet or mandrin 5 having a fixed position.

The particularity of the invention resides in the fact that there are provided automatic means for the advancement of the trocar cannula 4 and therefore of the slide 3 that supports the cannula.

The automatic means are constituted by return springs 10, which act between lateral pins 11 formed on the slide 3 and end pins 12 fixed on the inside wall of the body 2.

In order to load the springs, there is provided an actuation shaft 20 which protrudes from the body 2 on the opposite side with respect to the trocar cannula 4 and is provided with a folded edge 21, which engages the recess 22 formed on the lower face of the slide.

In order to retain the slide in loaded position, ratchet means are also provided, constituted by a detent spring 30, shown more clearly in figure 6, which has a flap 31 for fixing to an inside wall 32 of the body 2 and an engagement edge 33 constituted by a rectangular notch, provided so as to couple and lock with respect to the transverse recesses 23 formed by the recess 22, which in practice form a rack-like element.

The detent spring also has an inclined edge 34 which in practice connects the notch 33 to the tab and allows ratchet-like engagement with the transverse recesses 23 during loading.

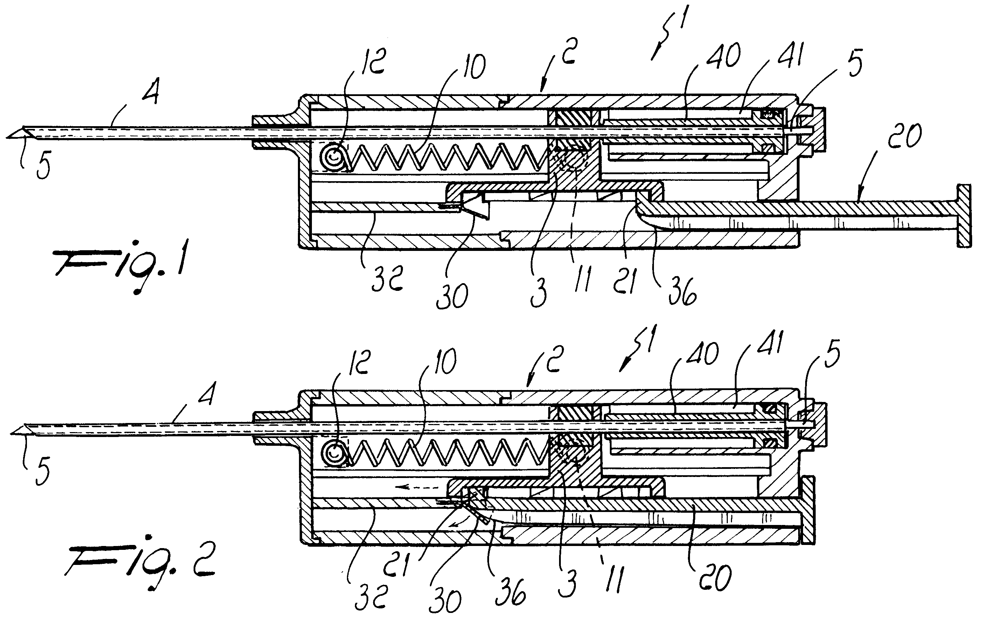

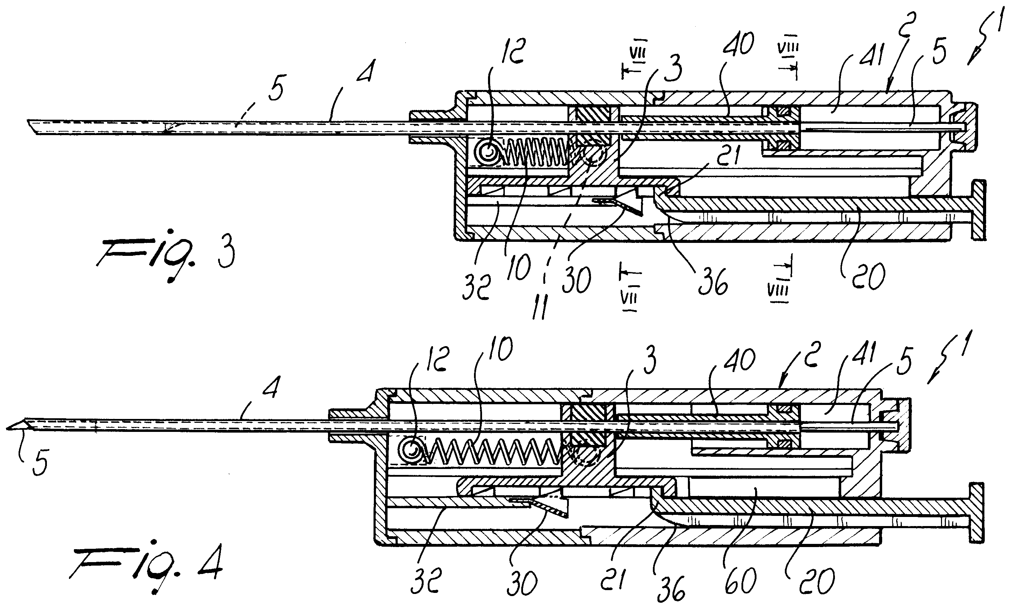

The spring 30 also has a central portion 35 with which the inclined end 36 of the actuation shaft 20 engages in order to uncouple the detent 30 from the transverse recesses 23, as shown in figure 2.

A plunger 40 is rigidly coupled to the cannula 4 and is sealingly movable in a cylindrical chamber 41 formed inside the body 2, so that when the slide 3 performs its forward translatory motion, moved by the springs 10, a suction effect is obtained because of the sliding of the plunger 40 in the cylindrical chamber 41.

An interspace is formed between the cannula 4 that supports the plunger 40 and the mandrin or stylet 5 that passes inside it, allowing the passage of the negative pressure, so as to produce suction at the front end of the trocar cannula; this suction in practice excises the tissue to remove the sample.

In practical operation, first the trocar cannula 4 is moved backwards with reference to the drawings, as shown in figure 1, by means of the actuation lever 20, so as to load the elastic means 10 and place the stylet 5, which protrudes axially from the cylindrical chamber 41, at the end of the cannula, so as to provide a puncturing point.

Once the device is in this condition, the cannula and the stylet are inserted at the region where the sample is to be removed, optionally with the aid of an ultrasonoscopic system that allows to exactly position the removal apparatus.

Once the chosen point has been reached, in order to perform all the removal steps it is sufficient to apply a slight pressure on the shaft 20 which, by means of the presence of the inclined portion 36, causes the lowering of the detent spring 30 and accordingly its disengagement from the transverse recesses 23, so that the slide 3, which was locked in position before, is free to move under the action of the springs 10.

During this translatory motion step, the trocar cannula moves with respect to the stylet, and during this translatory motion step, by moving the plunger element 40 which is rigidly coupled thereto, it produces a suction in the cylindrical chamber 41 which is transferred, by means of the previously mentioned interspace, at the end of the cannula, so that removal is performed automatically because the suction retains the sample, which is ultimately broken off at its base from the biopsied organ.

In order to expel the sample, it is sufficient, after removing the needle, to reload the device, as mentioned earlier, causing the consequent retraction of the cannula with respect to the removal needle and the expulsion of the sample.

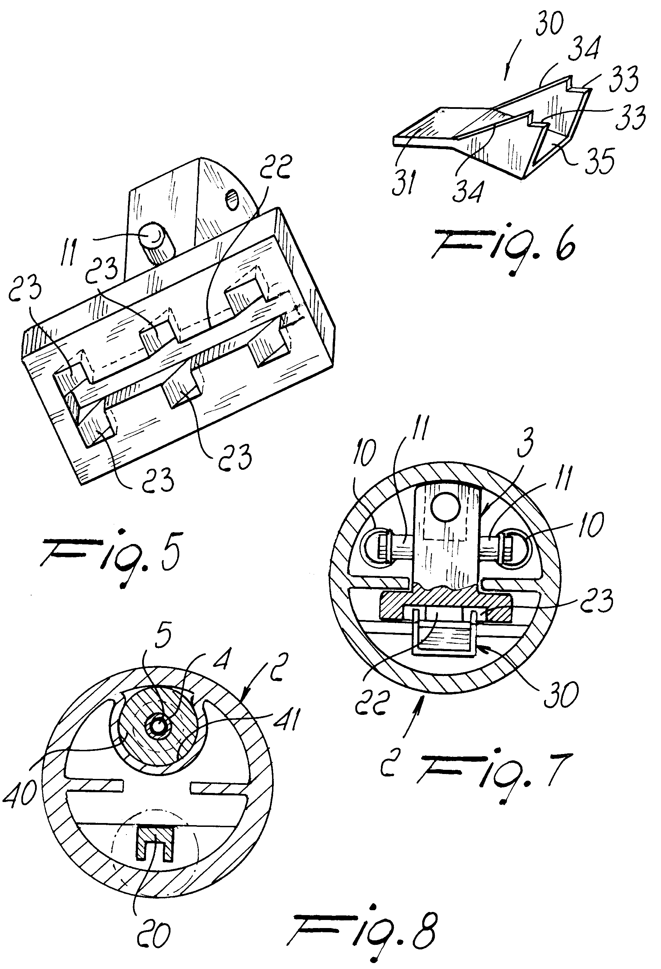

Figure 4 illustrates a different embodiment in which it is possible to perform a shorter removal stroke.

The shorter removal stroke can be obtained because of the fact that a plurality of mutually spaced transverse recesses are provided on the slide 3 and accordingly it is possible to initially lock the slide in various positions. In order to facilitate positioning, as clearly shown in figure 4, it is possible to provide spacers 60 which prevent the execution of longer strokes of the slide.

It is furthermore necessary to provide a stylet 5 of adequate length or to provide means which allow to adjust the useful length of the stylet 5 as a function of the amount to be removed, since, when the needle is inserted, the stylet 5 must always be located at the end of the trocar cannula 4.

From the above description it is evident that the invention achieves the intended aim and objects, and particularly the fact is stressed that the device according to the invention avoids the need to perform manual maneuvers while the needle is inserted in the patient's body, since it is sufficient to act on the spring 30, by means of the element 20, in order to release the slide 3 and accordingly produce the automatic sequence of all the movements, from the translatory motion of the cannula to the generation of the suction.

The invention thus conceived is susceptible of numerous modifications and variations, all of which are within the scope of the inventive concept.

All the details may furthermore be replaced with other technically equivalent elements.

In practice, the materials employed, as well as the contingent shapes and dimensions, may be any according to requirements.

Where technical features mentioned in any claim are followed by reference signs, those reference signs have been included for the sole purpose of increasing the intelligibility of the claims and accordingly, such reference signs do not have any limiting effect on the interpretation of each element identified by way of example by such reference signs.

## Claims

1. A device for taking biopsy samples, particularly of soft tissues, comprising a body which supports a trocar cannula inside which a fixed stylet is provided, characterized in that it comprises automatic means for the advancement of said trocar cannula, which is associated with a syringe-like element to produce suction inside said trocar cannula during the forward translatory motion with respect to said fixed stylet.

2. A device according to claim 1, characterized in that it comprises a slide which is slidingly movable inside said body and supports said trocar cannula, said stylet being rigidly fixed inside said body.

3. A device according to claim 2, characterized in that said automatic means for the advancement of said trocar cannula are constituted by elastic means provided by means of return springs which act between said slide and a fixed point of said body.

4. A device according to claim 3, characterized in that it comprises means for the disengageable locking of said slide, said means being constituted by ratchet means provided by a detent spring, which is supported by said body and has an engagement edge adapted to couple to one of a plurality of transverse recesses formed on the lower face of said slide, said transverse recesses providing a rack-like element for engagement with said ratchet means in positions which can be preset according to the length of the sample that can be produced.

5. A device according to claim 4, characterized in that it comprises means for loading said return springs, said means being constituted by an actuation shaft which protrudes from said body and has a folded edge for engagement with said slide.

6. A device according to claim 5, characterized in that said actuation shaft has, at one end, an inclined portion adapted to engage a central portion of said detent spring to disengage it from said transverse recesses.

7. A device according to claim 1, characterized in that it comprises a plunger which is rigidly associated with said trocar cannula and is sealingly movable in a cylindrical chamber formed inside said body, said cylindrical chamber being connected to an interspace formed between said stylet and said trocar cannula.

8. A device according to claim 1, characterized in that it comprises shim elements which can be inserted in said body to limit the stroke of said slide during loading.

9. A device according to claim 1, characterized in that it comprises means for adjusting the useful length of said stylet which can be actuated according to the stroke that can be performed by said trocar cannula.
